# EUROPEAN PATENT APPLICATION

(11) **EP 0 728 478 A1**
(43) Date of publication of application: **28.08.1996**
(21) Application number: 95900291.6
(22) Date of filing: 11.11.1994
(51) Int. Cl.: A61K 31/12, A61K 7/48, A61K 7/00

(54) **ANTIMICROBIAL METHOD AND COSMETIC COMPOSITION**

(30) Priority: 12.11.1993 JP 283069/93; 20.12.1993 JP 319409/93
(71) Applicant: OTSUKA PHARMACEUTICAL CO., LTD., Chiyoda-ku Tokyo 101 (JP)
(72) Inventor: NISHINO, Takeshi, 5-1, Sandanosa-cho Matsugasaki, Kyoto 606 (JP); OTSU, Yoshiro, Osaka 562 (JP); ARIMA, Yaeno, Kobe-shi Hyogo 654-01 (JP); NAKAI, Yoriko, Hyogo 669-33 (JP)
(74) Representative: Hansen, Bernd, Dr. Dipl.-Chem.
(86) International application number: JP9401911
(87) International publication number: WO9513057

(57) **Abstract**

An object of the present invention is to provide an antimicrobial method using a composition which is excellent in antimicrobial activity and light stability, low in toxicity and very weak in side effects.

The composition for use in the antimicrobial method of the present invention comprises a copper compound and at least one member selected from hinokitiol and salts thereof, or comprises at least one member selected from hinokitiol-copper or zinc complex and salts thereof.

## Description

### TECHNICAL FIELD

The present invention relates to antimicrobial methods and cosmetic compositions.

### BACKGROUND ART

Hinokitiol is conventionally used as an antibacterial agent for medicines, cosmetics and the like, but has the drawback of instability to ultraviolet rays. Therefore, it has been demanded to develop a still more improved antimicrobial agent.

### DISCLOSURE OF THE PRESENT INVENTION

In view of the above state of the art, the present inventors conducted extensive investigations in an attempt to develop a composition having an excellent antimicrobial activity, and consequently found that a composition comprising a copper compound and at least one member selected from hinokitiol and salts thereof and a composition comprising at least one member selected from hinokitiol-copper or zinc complexes and salts thereof are excellent not only in antimicrobial activity but also in light stability, in particular stability to ultraviolet rays. The present invention has been accomplished based on these findings.

Thus, the present invention provides an antimicrobial agent containing a copper compound and at least one member selected from hinokitiol and salts thereof, and an antimicrobial agent containing at least one member selected from hinokitiol-copper or zinc complexes and salts thereof.

The hinokitiol-copper complex and salts thereof for use as active ingredient(s) of the antimicrobial agent of the invention are known compounds. When a copper compound is mixed with at least one member selected from hinokitiol and salts thereof, hinokitiol-copper complex or a salt thereof may be formed. In this case, the obtained mixture can be used as an active ingredient for the invention without isolation and purification of the hinokitiol-copper complex or the salt.

The copper compound for use in the invention is not limited specifically and includes a wide variety of copper compounds conventionally known. Examples are copper (I) chloride, copper (II) acetate, copper (II) ammonium chloride, copper (I) bromide, copper (II) bromide, copper (I) bromide-dimethyl sulfide complex, copper (II) carbonate, copper (II) citrate, copper (I) cyanide, copper 4-cyclohexylbutyrate, copper (II) diammonium chloride, copper (II) diphosphate, copper (II) fluoride, copper (II) formate, copper (II) gluconate, copper (II) hydroxide, copper (I) iodide, copper naphthenate, copper (II) nitrate, copper (II) oleate, copper (II) oxalate, copper (II) oxide, copper (II) phosphate, copper (II) phthalate, copper (II) potassium chloride, copper (I) rhodanide, copper (II) sulfate, copper (II) sulfide, copper (I) thiocyanate, copper chlorophyll, hinokitiolato copper, copper nicotinate, nicotinamide copper compounds, copper picolinate, picolinamide copper compounds, copper salts and copper complexes of a compound selected from amino acids and peptides, and the like. In the practice of the invention, these copper compounds can be used either singly or in combination as a mixture of two or more.

Examples of the salts of hinokitiol to be used in the practice of the invention are inorganic salts including alkali metal salts such as sodium salt and potassium salt, alkaline earth metal salts such as magnesium salt and other metal salts such as copper salt and zinc salt, and organic salts including alkanolamine salts such as diethanolamine salt, 2-amino-2-ethyl-1,3-propanediol salt and triethanolamine salt, heterocyclic amine salts such as morpholine salt, piperazine salt and piperidine salt, ammonium salt, and basic amino acid salts such as arginine salt, lysine salt and histidine salt. The basic amino acids each may be in the D form or in the L form or in the form of a mixture of these. In the practice of the invention, hinokitiol and salts thereof may be used either singly or in combination as a mixture of two or more.

The proportions of the copper compound and hinokitiol or a salt thereof to be incorporated into the compositions of the invention are not particularly limited provided that the intended effects of the invention can be produced. However, it is recommended to use the copper compound in an amount of about 0.05 to 99.95% by weight, preferably about 10 to 99.95% by weight, most preferably about 50 to 99.9% by weight, and to use hinokitiol or a salt thereof in an amount of about 99.95 to 0.05 % by weight, preferably about 90 to 0.05% by weight, most preferably about 50 to 0.1% by weight, based on the total amount of the copper compound and hinokitiol or the salt thereof.

Hinokitiol-zinc complex and salts thereof for use as active ingredient(s) of the antimicrobial agent of the invention are known compounds. The proportions of hinokitiol and zinc are not limited specifically, but especially preferable are the former:latter ratios of 2:1 and 3:1.

Examples of the salts of hinokitiol-zinc complex to be used in the practice of the invention are inorganic salts including alkali metal salts such as sodium salt and potassium salt, alkaline earth metal salts such as magnesium salt and other metal salts such as copper salt and zinc salt, and organic salts including alkanolamine salts such as diethanolamine salt, 2-amino-2-ethyl-1,3-propanediol salt and triethanolamine salt, heterocyclic amine salts such as morpholine salt, piperazine salt and piperidine salt, ammonium salt, and basic amino acid salts such as arginine salt, lysine salt and histidine salt, and inorganic or organic acid salts such as sulfuric acid, hydrochloric acid, acetic acid, citric acid and lactic acid. The basic amino acids each may be in the D form or in the L form or in the form of a mixture of these. In the practice of the invention, hinokitiol-zinc complex and salts thereof may be used either singly or in combination as a mixture of two or more.

When the compositions of the invention are to be used as medicaments, the copper compound and hinokitiol or a salt thereof may be used in such a manner that both of them are contained in a single pharmaceutical preparation. It is also possible to formulate them independently into separate preparations and utilize both of the pharmaceutical preparations. Hinokitiol-copper complex or salts thereof and/or, hinokitiol-zinc complex or salts thereof can be used in such a manner that each of them is contained in a single pharmaceutical preparation. Such preparations are produced by using diluents or excipients that are generally used, including fillers, extendering agents, binders, humectants, disintegrating agents, surfactants, lubricants and so on. For these pharmaceutical preparations, various forms can be selected depending on the purpose of therapy and, as typical examples thereof, there may be mentioned tablets, pills, powders, solutions, suspensions, emulsions, granules, capsules, suppositories, injections (solutions, suspensions, etc.), oleaginous ointments, emulsion ointments, water-soluble ointments, pastes, plasters, lotions, liniments and other preparations for external use.

When the compositions of the invention are to be used as cosmetics, they can be added to the cosmetics as antidandruff agents, anti-acne agents, antiperspirants and deodorants, antifungal agents, bactericides and/or preservatives for preserving the cosmetics, for instance. Said cosmetics may be used in various forms including skin care products such as cleansing preparations, creams, milk lotions, makeup creams, cosmetic oils, packs or the like; makeup cosmetics such as foundations, lipsticks, cheek rouges, eyeliners, mascaras, eye shadows, manicures, face powders or the like; hair care preparations such as hair dressing preparations, hair tonics, hair dye or the like; oral sanitary preparations such as dentifrice, mouth wash or the like; bath preparations, whitening preparations, sunscreen preparations, acne treatment preparations, etc. These can be produced by the methods that are conventional in the art.

In producing such cosmetics, various known cosmetic vehicle materials, such as vehicles, binders, lubricants, disintegrating agents, etc., can be used as necessary and, further, various oleaginous materials such as oils and fats, waxes, hydrocarbons, fatty acids, higher alcohols, ester oils, metal soaps, etc., pharmacologically active materials such as animal or plant extracts, vitamins, hormones, amino acids, etc., surfactants, coloring matter, dyes, pigments, perfumes, preservatives, bactericides, humectants, thickening agents, antioxidants, sequestering agents and other various components and additives already known can be used as necessary in a suitable combination.

In using the compositions of the invention as medicaments, the active ingredient concentrations are not particularly limited provided that the compositions have the desired effects or benefits. Generally, however, they recommendably contain a copper compound and hinokitiol or a salt thereof in a total amount of about 0.001 to 20% by weight, preferably about 0.01 to 10% by weight. When the compositions of the invention are made into preparations to be used as diluted, for example preparations to be adjusted immediately prior to use, the preparations generally contain the copper compound and hinokitiol or a salt thereof in a total amount of about 0.001 to 100% by weight, preferably about 0.001 to 50% by weight. Likewise, when using hinokitiol-copper complex or a salt thereof, the compositions of the invention generally contain said complex or a salt thereof in an amount of about 0.001 to 20% by weight, preferably about 0.01 to 10% by weight. When the compositions are made into preparations to be used as diluted, for example preparations to be adjusted immediately prior to use, the preparations generally contain said complex or a salt thereof in an amount of about 0.001 to 100% by weight, preferably about 0.001 to 50% by weight. When using hinokitiol-zinc complex or a salt thereof, the compositions of the invention also contain said complex or a salt thereof generally in an amount of about 0.001 to 20% by weight, preferably about 0.01 to 10% by weight. When the compositions are made into preparations to be used as diluted, for example preparations to be adjusted immediately prior to use, the preparations generally contain said complex or a salt thereof in an amount of about 0.001 to 100% by weight, preferably about 0.001 to 50% by weight.

When the compositions of the invention are to be used as cosmetics, the active ingredient concentrations may vary depending on the form and the like, hence cannot be specified in a general manner or are not particularly limited. Generally, however, the compositions recommendably contain a copper compound and hinokitiol or a salt thereof in total, or a hinokitiol-copper or zinc complex or a salt thereof, in an amount of about 0.0001 to 99.9% by weight, preferably about 0.001 to 30% by weight. The cosmetics mentioned above may be used after further dilution with water, ethanol, olive oil, liquid gas or some other suitable solvents.

In shaping into the form of tablets, a variety of materials so far well known as vehicles in this field of art can be used. Thus, for example, use may be made of excipients such as lactose, sucrose, sodium chloride, glucose, urea, starch, calcium carbonate, kaolin, crystalline cellulose, silicic acid, etc., binders such as water, ethanol, propanol, simple syrup, glucose solution, starch solution, gelatin solution, carboxymethylcellulose, shellac, methyl cellulose, potassium phosphate, polyvinylpyrrolidone, etc., disintegrating agents such as dried starch, sodium alginate, agar powder, laminaran powder, sodium hydrogen carbonate, calcium carbonate, polyoxyethylenesorbitan fatty acid esters, sodium lauryl sulfate, stearic acid monoglyceride, starch, lactose, etc., disintegration preventing agents such as sucrose, stearin, cacao butter, hydrogenated oils, etc., absorption promoting agents such as quaternary ammonium bases, sodium lauryl sulfate, etc., humectants such as glycerin, starch, etc., adsorbents such as starch, lactose, kaolin, bentonite, colloidal silicic acid, etc., lubricants such as purified talc, corn starch, waxes, polyethylene glycol, etc., and so forth. When necessary, the tablets may further be processed into tablets coated with an ordinary coating film, for example sugar coated tablets, gelatin coated tablets, enteric coated tablets, film coated tablets, or double-layered or multilayered tablets.

In shaping into the form of pills, a wide variety of materials so far known as vehicles in this field can be used. Thus, for example, use may be made of excipients such as glucose, lactose, starch, cacao butter, hydrogenated vegetable oils, kaolin, talc, etc., binders such as powdered acacia, powdered tragacanth, gelatin, ethanol, etc., disintegrating agents such as laminaran, agar, etc., and so forth.

In shaping into the form of suppositories, a wide variety of materials so far known as vehicles in this field can be used. Thus, for example, there may be mentioned polyethylene glycol, cacao butter, higher alcohols, higher alcohol esters, gelatin, semisynthetic glycerides, and so on.

In preparing injections, the solutions, emulsions and suspensions are sterilized and are preferably isotonic with blood. For producing these forms, all diluents conventionally used in this field can be employed. Thus, for instance, water, ethyl alcohol, macrogols, propylene glycol, ethoxylated isostearyl alcohol, polyoxylated isostearyl alcohol, polyoxyethylene sorbitan fatty acid esters and the like can be used. In this case, sodium chloride, glucose or glycerin may be incorporated into the pharmaceutical preparations in an amount sufficient to give isotonic solutions. Conventional solubilizing agents, buffers, soothing agents and the like may also be added.

In preparing ointments, a wide variety of oleaginous bases so far known in this field can be used. As specific examples, there may be mentioned oils and fats such as peanut oil, sesame oil, soybean oil, safflower oil, avocado oil, sunflower oil, corn oil, rape seed oil, cottonseed oil, castor oil, camellia oil, coconut oil, olive oil, poppy seed oil, cacao oil, beef tallow, lard, wool oil, etc., mineral oils such as vaseline, paraffin, silicone oil, squalane, etc., higher fatty acid esters, higher aliphatic alcohols and waxes such as isopropyl myristate, ceyl isooctanoate, n-butyl myristate, isopropyl linoleate, propyl ricinoleate, isopropyl ricinoleate, isobutyl ricinoleate, heptyl ricinoleate, diethyl sebacate, diisopropyl adipate, cetyl alcohol, stearyl alcohol, behenyl alcohol, batyl alcohol, chimyl alcohol, white beeswax, spermaceti, Japan wax, etc, higher fatty acids such as stearic acid, oleic acid, palmitic acid, etc., mono-, di- and triglyceride mixtures derived from saturated or unsaturated fatty acids containing 12 to 18 carbon atoms, and so on. In the practice of the invention, these bases may be used either singly or in combination as a mixture of two or more.

Conventional additives, for example metal soaps, animal or plant extracts, vitamins, hormones, amine acids or other pharmacologically active substances, surfactants, coloring matter, dyes, pigments, perfumes, ultraviolet absorbers, humectants, thickening agents, antioxidants, sequestering agents, pH regulators, etc., may be incorporated, as necessary, into the compositions of the invention.

The compositions of the invention are produced by a conventional method.

The method of administration of the above-mentioned pharmaceutical preparations is not particularly limited but the preparations are administered by a method suitably selected depending on the dosage form, the age, sex and other conditions of the patient, the severity of the disease and other factors. Thus, tablets, pills, solutions, suspensions, emulsions, granules and capsules, for instance, are administered orally. Injections are administered, either as such or in admixture with an ordinary parenteral fluid such as glucose, amino acid or the like, by the intravenous route. If desired, they are administered as such via intramuscular, intradermal, subcutaneous or intraperitoneal route. Suppositories are administered rectally. External preparations are applied to the affected parts.

These pharmaceutical preparations of the present invention are recommendably administered in such an amount that the amount of the active ingredient(s), i.e., a copper compound and hinokitiol or a salt thereof in total, or a hinokitiol-copper or zinc complex or a salt thereof, is about 1 to 20 mg/kg body weight per human adult per day, and the preparations are administered in a single dose or two or three divided doses.

In using the compositions of the invention in the field of sterilization for external use, the proportions of the copper compound and hinokitiol or a salt thereof in the compositions are not particularly limited provided that the compositions produce the desired effects. However, it is recommended that the compositions of the invention contain the copper compound generally in an amount of about 0.00005 to 99% by weight, preferably about 0.001 to 30% by weight, and hinokitiol or a salt thereof generally in an amount of about 0.00005 to 50% by weight, preferably about 0.001 to 20% by weight. When using hinokitiol-copper complex, the compositions of the invention contain said complex generally in an amount of about 0.0001 to 60% by weight, preferably about 0.01 to 30% by weight. When using hinokitiol-zinc complex, the compositions of the invention contain said complex generally in an amount of about 0.0001 to 60% by weight, preferably about 0.01 to 30% by weight. The compositions of the invention may also be diluted for external application, if so desired.

The compositions of the invention for sterilization for external use may contain various additives conventionally known in this field in addition to the above-mentioned active ingredients. Examples of such additives are fats and oils such as avocado oil, olive oil, coconut oil, beef tallow, lard, etc.; waxes such as beeswax, spermaceti, lanolin, etc; hydrocarbons such as liquid paraffin, vaseline, squalane, silicone oil, etc.; fatty acids such as lauric acid, myristic acid, palmitic acid, stearic acid, oleic acid, etc.; higher aliphatic alcohols such as cetyl alcohol, stearyl alcohol, 2-hexyldecanol, 2-octyldodecanol, etc.; esters such as isopropyl myristate, 2-octyldodecyl myristate, cetyl isooctanoate, isononyl isononylate, dinonylpropylene glycol, etc.; mono-, di- and triglycerides of saturated or unsaturated fatty acids containing 8 to 20 carbon atoms such as glycerol monostearate, glycerol monooleate, etc.; lower alcohols such as methanol, ethanol, isopropanol, etc.; polyhydric alcohols such as glycerin, propylene glycol, 1,3-butylene glycol, hexylene glycol, etc.; water; various organic solvents; metal soaps; pharmacologically active materials such as animal or plant extracts, vitamins, hormones, amines, etc.; surfactants, coloring matter, dyes, pigments, perfumes, ultraviolet absorbers, humectants, thickening agents, antioxidants, sequestering agents, pH regulators, skin astringents, deodorants, bleaches, fluorescent whitening agents, builders, various gases for aerosol, etc.

In the practice of the method of the invention, conventional means can be used for applying the composition of the invention to the part to be externally sterilized. Examples of the means include application by spraying, application by brushing, wiping with a cloth, a paper towel or wet tissues, aerosol, etc.

The composition of the invention can be added to cleaning agents, starches, softeners, bleaches and the like for proprietary and medical supply to be externally sterilized (such as clothes, food, furniture, gauze, sheets, curtains, etc.). The compositions of the invention are also suitably applied to animals and products for animals to be externally sterilized.

In addition, the compositions of the invention can be added to foods as a preservative.

The compositions of the invention have an excellent antimicrobial activity and are particularly useful for the treatment of various infectious diseases caused by various microorganisms, for example various skin diseases including dermatitis such as atopic dermatitis, nummular dermatitis, autosensitization dermatitis, diaper dermatitis and stasis dermatitis, eczema such as housewives hand eczema and dry eczema, pustular psoriasis, burn, redness resulting from burn, impetigo, rash, miliaria, sore, abrasion, secondary infection through scratching, etc.

In addition, the compositions of the invention are very low in the irritation and allergy effect on the skin and therefore can be suitably applied not only to babies, infants, children and other patients with skin diseases, for example, patients susceptible to skin irritation, but also in the treatment of skin diseases in mammals other than humans (pet animals such as dogs, cats, etc., domestic animals such as cattle, horses, etc., and so on).

The present composition comprising a copper compound and hinokitiol or a salt thereof as active ingredients and the present composition comprising hinokitiol-copper complex or a salt thereof as an active ingredient are excellent in antimicrobial activity, stable to light such as ultraviolet rays, and low in irritancy, and thus can be suitably used in babies, infants, children and other patients with skin diseases, patients susceptible to irritation and so forth. These compositions of the invention can also be suitably used in patients with atopic dermatitis or contact dermatitis.

The composition of the invention comprising hinokitiol-zinc complex or a salt thereof as active ingredient(s) has an excellent antimicrobial activity, especially antibacterial (not antifungal) activity and excellent stability, and is durable, readily absorbed, highly safe, and low in skin irritancy. Therefore, said composition of the invention can be suitably used in babies, infants, children and other patients with skin diseases, patients susceptible to irritation and so forth. Said composition of the invention can also be suitably used in patients with atopic dermatitis or contact dermatitis, and exhibits a remarkable effect especially as an antidandruff agent.

The compositions of the invention have excellent antimicrobial activities against a wide variety of gram-positive and gram-negative bacteria and are low in toxicity and very low in side effects. In particular, said compositions have very strong antimicrobial activities against gram-positive bacteria such as those belonging to the genera Staphylococcus, Streptococcus, Corynebacterium diphtheriae, Mycobacterium tuberculosis and Clostridium, and gram-negative bacteria such as those belonging to the genera Neisseria, Escherichia, Citrobacter, Salmonella, Shigella, Klebsiella and Pseudomonas, anaerobes such as those belonging to the genera Clostridium, Pentococcus, Peptostreptococcus, Propionibacterium and Bacteroides, Zygomycetes including those belonging to the genus Mucor, Ascomycetes including those belonging to the genera Aspergillus and Penicillium, Deuteromycotina including those belonging to the genera Cryptococcus, Candida, Dermatophytes and Pityrosporum, and like fungi. The compositions of the invention exhibit an excellent antimicrobial activity against clinical strains, as well as standard strains.

The compositions of the invention exhibit excellent antimicrobial activities against various bacteria and clinical isolates, which are resistant to penicillins, cephems, quinolones, aminoglycosides, macrolides, tetracyclines and like antibiotics. In particular, the compositions of the invention produce remarkable antimicrobial activities against MRSA and MRSE. Furthermore, the compositions of the invention are efficacious against multiple drug-resistant MRSA and MRSE such as those resistant to quinolone, aminoglycoside, macrolide and tetracycline antibiotics.

The use of hinokitiol as a complex with copper or as a complex with zinc, or the use of a copper compound in combination with hinokitiol or a salt thereof inhibits the paradoxical effect observed when hinokitiol or a salt thereof is singly used against genus Staphylococcus or the like, and therefore the bactericidal effects of hinokitiol or a salt thereof is further increased.

Accordingly, the compositions of the invention are advantageously applicable for cleaning wet tissues, chemical clothes, paper towels, moist hand towels, carpets, floors, tableware, general clothing, general instruments (such as furniture and glasses), medical clothing, etc., for removing bacteria from dishwashers, drainpipes, etc. and for washing and removing bacteria from medical instruments (such as gauze, optical lenses, etc.), beverage containers, products for animals, etc.

Hinokitiol, when placed in a polyethylene container conventionally used, adheres to or is adsorbed by the polyethylene container, and thereby the antibacterial effect of hinokitiol is reduced. Contrastingly, the hinokitiol-copper or zinc complex and salts thereof for use in the invention can exhibit excellent antibacterial effects without adhering to nor being absorbed by the polyethylene container.

### BEST MODE FOR CARRYING OUT THE INVENTION

The following reference examples, production examples and test examples are further illustrative of the present invention. Hereinafter, "%" means "% by weight".

### Reference Example

Reagents used were hinokitiol manufactured by Takasago Perfumery Co., Ltd., and zinc acetate dihydrate and ethanol both of which are guaranteed reagent of Wako Pure Chemical Industries, Ltd. All the drugs were used without any treatment.

A 5.0 g quantity of hinokitiol was dissolved in 200 ml of ethanol with stirring. In the solution was further dissolved 3.4 g of zinc acetate dihydrate. The solution was stirred for 5 hours, and the precipitate was filtered with a No. 5C paper filter and dried under reduced pressure using a vacuum pump (VACUUM PUMP 4VP-C₄, product of Hitachi Ltd.) to obtain 4.6 g of dihinokitiolato zinc (II) represented by the following formula.

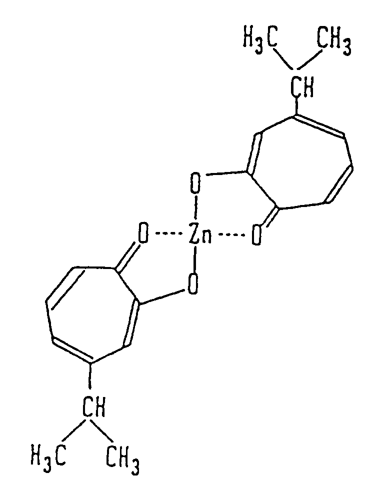

### Production Example 1 (Zinc oxide ointment)

| | |
|---|---|
| Hinokitiol | 0.5 g |
| Lard | 300.0 g |
| Bleached beeswax | 60.0 g |
| Zinc oxide | 100.0 g |
| Copper (II) oxide | 0.1 g |
| White petrolatum | suitable amount |
| Total | 1000.0 g |

(1) Hinokitiol and copper (II) oxide were weighed, and a portion of lard was added thereto. The mixture was heated to about 40°C and stirred to give a homogeneous melt. (2) The remaining portion of lard, bleached beeswax and white petrolatum were dissolved on a water bath and stirred to give a mixture at 80°C. (3) Zinc oxide was weighed and placed in a mortar. The mixture prepared in the above step (2) was added portionwise with stirring to give a homogenous mixture, which was then cooled to about 40°C with stirring. (4) Then, the mixture prepared in the above step (1) was added at about 40°C, and the resulting mixture was fully stirred until solidification to give the desired ointment.

### Production Example 2 (Ointment)

| | |
|---|---|
| Hinokitiol | 0.1 g |
| Olive oil | 100.0 g |
| Copper (II) oxide | 100.0 g |
| Simple ointment (Japanese Pharmacopoeia) | suitable amount |
| Total | 1000.0 g |

(1) Hinokitiol was weighed, and a portion of olive oil was added thereto. The mixture was heated to about 40°C and stirred to give a homogeneous melt. (2) The remaining portion of olive oil and simple ointment (Japanese Pharmacopoeia) were dissolved on a water bath and then stirred to give a mixture at 80°C. (3) Copper (II) oxide was weighed and placed in a mortar. The mixture prepared in the above step (2) was added portionwise with stirring to give a homogenous mixture, which was then cooled to about 40°C with stirring. (4) Then, the mixture prepared in the above step (1) was added at about 40°C, and the resulting mixture was fully stirred until solidification to give the desired ointment.

### Production Example 3 (Ointment)

| | |
|---|---|
| Lipophilic glycerin monostearate | 50.0 g |
| Copper (II) oxide | 50.0 g |
| White petrolatum | suitable amount |
| Hinokitiol sodium salt | 0.05 g |
| Glycerin | 30.0 g |
| Purified water | 50.0 g |
| Total | 1000.0 g |

(1) Hinokitiol sodium salt, glycerin and purified water were heated to about 60°C and mixed with stirring to give a homogeneous melt. (2) Lipophilic glycerin monostearate and white petrolatum were dissolved on a water bath and then stirred to give a mixture at 60°C. (3) The mixture prepared in the above step (1) was added portionwise to the mixture prepared in the above step (2) with uniform stirring. The whole mixture was then cooled to about 40°C with stirring. (4) Copper (II) oxide was weighed and placed in a mortar. The mixture prepared in the above step (3) was added portionwise, and the resulting mixture was fully stirred until solidification to give the desired ointment.

### Production Example 4 (Vanishing cream)

| | |
|---|---|
| Stearic acid | 10.0% |
| Paraffin wax (135F) | 2.0% |
| Spermaceti | 2.0% |
| Cetyl alcohol | 2.0% |
| Cetyl isooctanoate | 5.0% |
| Polyoxyethylene (20EO) sorbitan monolaurate | 3.0% |
| Copper (II) acetate | 0.015% |
| Sodium hydroxide | 0.15% |
| Concentrated glycerin | 5.0% |
| Hinokitiol | 0.02% |
| Perfume | suitable amount |
| Purified water | Balance |
| Total | 100.0% |

(1) Stearic acid, paraffin wax (135F), spermaceti, cetyl alcohol, cetyl isooctanoate and polyoxyethylene (20EO) sorbitan monolaurate were heated to 80-85°C and stirred to give a homogeneous melt. (2) Copper (II) acetate, sodium hydroxide, concentrated glycerin and purified water were heated to 80-85°C and stirred to give an uniform solution. (3) At 80°C, the mixture prepared in the above step (2) was added portionwise to the mixture prepared in the above step (1) and, after uniform emulsification, the whole mixture was cooled to 45°C with stirring. (4) At 45°C, hinokitiol and perfume were added to the mixture prepared in the above step (3), and the resulting mixture was stirred to homogeneity and cooled to room temperature with stirring to give the desired vanishing cream.

### Production Example 5 (Cleansing cream)

| | |
|---|---|
| Bleached beeswax | 3.0% |
| Liquid paraffin | 30.0% |
| Cetyl alcohol | 2.0% |
| Cetyl isooctanoate | 10.0% |
| Triethanolamine | 0.2% |
| Propylene glycol | 5.0% |
| Copper (II) acetate | 0.1% |
| Antioxidant | suitable amount |
| Hinokitiol | 0.05% |
| Perfume | suitable amount |
| Purified water | Balance |
| Total | 100.0% |

Following the procedure of Production Example 4, the above ingredients were admixed and stirred with optional heating to give a cleansing cream.

### Production Example 6 (Lotion)

| | |
|---|---|
| Ethyl alcohol | 10.0% |
| Polyoxyethylene (9EO) lauryl ether | 2.0% |
| Kankoh SO 201 | 0.001% |
| Perfume | suitable amount |
| Concentrated glycerin | 5.0% |
| 1,3-Butylene glycol | 3.0% |
| Hinokitiol-copper complex | 0.05% |
| Hinokitiol | 0.05% |
| Color | suitable amount |
| Purified water | Balance |
| Total | 100.0% |

(1) Polyoxyethylene (9EO) lauryl ether, Kankoh SO 201, hinokitiol-copper complex, hinokitiol and perfume were added to ethyl alcohol, followed by uniform mixing.
(2) Concentrated glycerin and 1,3-butylene glycol were added to and uniformly dissolved in purified water. (3) At 60°C, the mixture prepared in the above step (2) was added to the mixture prepared in the above step (1) and, after uniform mixing, color was added for coloration to give a lotion.

### Production Example 7 (Ointment)

| | |
|---|---|
| Lard | 20.95% |
| Bleached beeswax | 7.0% |
| Lipophilic glycerin monostearate | 2.0% |
| White petrolatum | 60.0% |
| Copper (II) oxide | 10.0% |
| Hinokitiol | 0.05% |
| Total | 100.0% |

(1) Hinokitiol was weighed, and a portion of lard was added thereto. The mixture was heated to about 40°C and stirred to give a homogeneous melt. (2) The remaining portion of lard, lipophilic glycerin monostearate, bleached beeswax and white petrolatum were dissolved on a water bath and stirred to give a mixture at 80°C. (3) Copper (II) oxide was weighed and placed in a mortar. The mixture prepared in the above step (2) was added portionwise with uniform stirring. The resulting mixture was cooled to about 40°C with stirring.
(4) Then, the mixture prepared in the above step (1) was added at about 40°C. The whole mixture was fully stirred until solidification to give the desired ointment.

### Production Example 8 (Disinfectant for pumps)

| | |
|---|---|
| Hinokitiol | 0.05 g |
| Copper (I) chloride | 0.04 g |
| 99.5% Ethanol | 70.0 g |
| Purified water | Balance |
| Total | 100.0 g |

### Production Example 9 (Shampoo for pet animals)

| | |
|---|---|
| Sodium lauroyl methylalanine (30%) | 40.0 g |
| Cocoyl amidopropyldimethyl aminoacetic acid betaine | 10.0 g |
| Hinokitiol | 0.1 g |
| 99.5% Ethanol | 3.0 g |
| Copper (I) chloride | 0.08 g |
| pH adjusting agent | suitable amount |
| Purified water | Balance |
| Total | 100.0 g |

### Production Example 10 (Solid soap)

| | |
|---|---|
| Soap material | 30.0 g |
| Sodium hydrogenated cocomonoglyceride sulfate | 50.0 g |
| Glyceryl monostearate | 5.0 g |
| Cetyl alcohol | 5.0 g |
| Titanium dioxide | 0.5 g |
| Copper (II) oxide | 0.5 g |
| Hinokitiol | 0.05 g |
| Perfume, antioxidant and sequestering agent | suitable amount |

### Production Example 11 (Detergent for instruments)

| | |
|---|---|
| Sodium cocoyl methyl alaninate (25%) | 15.0 g |
| Cocoyl amidepropyldimethyl aminoacetic acid betaine | 5.0 g |
| Hinokitiol·triethanolamine salt | 0.01 g |
| Hinokitiol·L-arginine acid | 0.03 g |
| Copper (II) gluconate | 0.001 g |
| pH adjusting agent | suitable amount |
| Purified water | Balance |
| Total | 100.0 g |

### Production Example 12 (Liquid detergent for laundry)

| | |
|---|---|
| Polyoxyethylene lauryl ether | 35.0 g |
| Alkylbenzenesulfonic acid triethanolamine salt | 15.0 g |
| Hinokitiol·2-amino-2-methyl-1,3-propanediol salt | 0.5 g |
| Copper (I) chloride | 0.4 g |
| Purified water | Balance |
| Total | 100.0 g |

### Production Example 13 (Liquid detergent for laundry)

| | |
|---|---|
| Alkylbenzenesulfonic acid triethanolamine salt | 19.0 g |
| Polyoxyethylene dodecyl ether | 7.0 g |
| Sodium methaxylenesulfonate | 6.0 g |
| Triethanolamine citrate | 8.0 g |
| Carboxymethylcellulose | 0.5 g |
| Hinokitiol·2-amino-2-methyl-1,3-propanediol salt | 1.0 g |
| Copper (II) citrate | 0.4 g |
| Fluorescent brightener and perfume | suitable amount |
| Purified water | Balance |
| Total | 100.0 g |

### Production Example 14 (Powdery detergent for laundry)

| | |
|---|---|
| Beef tallow soap | 45.0 g |
| Coconut oil soap | 10.0 g |
| Anhydrous sodium carbonate | 35.0 g |
| Anhydrous sodium sulfate | 7.0 g |
| Hinokitiol-copper complex | 0.5 g |
| Hinokitiol·sodium salt | 0.5 g |
| Fluorescent brightener and perfume | suitable amount |
| Purified water | Balance |
| Total | 100.0 g |

### Production Example 15 (Detergent for bathroom)

| | |
|---|---|
| Polyoxyethylene alkyl (C₁₂) ether | 10.0 g |
| Citric acid | 5.0 g |
| Polypropylene glycol | 5.0 g |
| 99.5 % Ethanol | 3.0 g |
| Hinokitiol | 0.05 g |
| Copper (II) acetate | 0.05 g |
| pH adjusting agent | suitable amount |
| Purified water | Balance |
| Total | 100.0 g |

### Production Example 16 (Detergent for toilet)

| | |
|---|---|
| Hydrochloric acid | 9.5 g |
| Sodium chloride | 7.0 g |
| Polyoxyethylene alkyl (C₁₂) ether | 2.0 g |
| Thickening agent | 2.5 g |
| Hinokitiol | 0.03 g |
| Copper (I) chloride | 0.5 g |
| pH adjusting agent | suitable amount |
| Purified water | Balance |
| Total | 100.0 g |

### Production Example 17 (Wiping agent, bulk for aerosol)

| | |
|---|---|
| Liquid paraffin | 0.3 g |
| Sucrose fatty acid ester | 2.0 g |
| 1,3-Butylene glycol | 3.0 g |
| Hinokitiol | 0.02 g |
| 99.5 % Ethanol | 5.0 g |
| Copper (I) chloride | 0.01 g |
| Purified water | Balance |
| Total | 100.0 g |

### Production Example 18 (Zinc oxide ointment)

| | |
|---|---|
| Hinokitiol-zinc complex | 0.5 g |
| Lard | 300.0 g |
| Bleached beeswax | 60.0 g |
| Zinc oxide | 100.0 g |
| White petrolatum | suitable amount |
| Total | 1000.0 g |

(1) Hinokitiol-zinc complex was weighed, and a portion of lard was added thereto. The mixture was heated to about 40°C and stirred to give a homogeneous melt. (2) The remaining portion of lard, bleached beeswax and white petrolatum were dissolved on a water bath and stirred to give a mixture at 80°C. (3) Zinc oxide was weighed and placed into a mortar. The mixture prepared in the above step (2) was added portionwise with stirring to give a homogenous mixture, which was then cooled to about 40°C with stirring. (4) Then, the mixture prepared in the above step (1) was added at about 40°C, and the resulting mixture was fully stirred until solidification to give the desired ointment.

### Production Example 19 (Ointment)

| | |
|---|---|
| Hinokitiol-zinc complex | 0.1 g |
| Olive oil | 100.0 g |
| Simple ointment (Japanese Pharmacopoeia) | suitable amount |
| Total | 1000.0 g |

(1) Hinokitiol-zinc complex was weighed, and a portion of olive oil was added thereto. The mixture was heated to about 40°C and stirred to give a homogeneous melt. (2) The remaining portion of olive oil and simple ointment (Japanese Pharmacopoeia) were dissolved on a water bath and then stirred to give a mixture at 80°C. (3) The mixture prepared in the above step (2) was gradually poured into a mortar with uniform stirring, and cooled to about 40°C with stirring. (4) Then, the mixture prepared in the above step (1) was added at about 40°C, and the resulting mixture was fully stirred until solidification to give the desired ointment.

### Production Example 20 (Ointment)

| | |
|---|---|
| Lipophilic glycerin monostearate | 50.0 g |
| White petrolatum | suitable amount |
| Sodium salt of hinokitiol-zinc complex | 0.05 g |
| Glycerin | 30.0 g |
| Purified water | 50.0 g |
| Total | 1000.0 g |

(1) Sodium salt of hinokitiol-zinc complex, glycerin and purified water were heated to about 60°C and stirred to give a homogeneous melt. (2) Lipophilic glycerin monostearate and white petrolatum were dissolved on a water bath and then stirred to give a mixture at 60°C. (3) The mixture prepared in the above step (1) was added portionwise to the mixture prepared in the above step (2) with uniform stirring. The whole mixture was then cooled to about 40°C with stirring. (4) The mixture prepared in the above step (3) was gradually poured into a mortar, and fully stirred until solidification to give the desired ointment.

### Production Example 21 (Vanishing cream)

| | |
|---|---|
| Stearic acid | 10.0% |
| Paraffin wax (135F) | 2.0% |
| Spermaceti | 2.0% |
| Cetyl alcohol | 2.0% |
| Cetyl isooctanoate | 5.0% |
| Polyoxyethylene (20EO) sorbitan monolaurate | 3.0% |
| Sodium hydroxide | 0.15% |
| Concentrated glycerin | 5.0% |
| Hinokitiol-zinc complex | 0.02% |
| Perfume | suitable amount |
| Purified water | Balance |
| Total | 100.0% |

(1) Stearic acid, paraffin wax (135F), spermaceti, cetyl alcohol, cetyl isooctanoate and polyoxyethylene (20EO) sorbitan monolaurate were heated to 80-85°C and stirred to give a homogeneous melt. (2) Sodium hydroxide, concentrated glycerin and purified water were heated to 80-85°C and stirred to give a homogeneous solution. (3) At 80°C, the mixture prepared in the above step (2) was added portionwise to the mixture prepared in the above step (1) and, after uniform emulsification, the whole mixture was cooled to 45°C with stirring. (4) At 45°C, hinokitiol-zinc complex and perfume were added to the mixture prepared in the above step (3), and the resulting mixture was uniformly stirred and cooled to room temperature with stirring to give the desired vanishing cream.

### Production Example 22 (Cleansing cream)

| | |
|---|---|
| Bleached beeswax | 3.0% |
| Liquid paraffin | 30.0% |
| Cetyl alcohol | 2.0% |
| Cetyl isooctanoate | 10.0% |
| Triethanolamine | 0.2% |
| Propylene glycol | 5.0% |
| Antioxidant | suitable amount |
| Hinokitiol-zinc complex | 0.05% |
| Perfume | suitable amount |
| Purified water | Balance |
| Total | 100.0% |

Following the procedure of Production Example 21, the above ingredients were admixed and stirred with optional heating to give the desired cleansing cream.

### Production Example 23 (Lotion)

| | |
|---|---|
| Ethyl alcohol | 10.0% |
| Polyoxyethylene (9EO) lauryl ether | 2.0% |
| Kankoh SO 201 | 0.001% |
| Perfume | suitable amount |
| Concentrated glycerin | 5.0% |
| 1,3-Butylene glycol | 3.0% |
| Hinokitiol-zinc complex | 0.05% |
| Color | suitable amount |
| Purified water | Balance |
| Total | 100.0% |

(1) Polyoxyethylene (9EO) lauryl ether, Kankoh SO 201, hinokitiol-zinc complex and perfume were added to and uniformly dissolved in ethyl alcohol. (2) Concentrated glycerin and 1,3-butylene glycol were added to and uniformly dissolved in purified water. (3) At 60°C, the mixture prepared in the above step (2) was added to the mixture prepared in the above step (1). The whole mixture was uniformly mixed, and color was added for coloration to give a lotion.

### Production Example 7 (Ointment)

| | |
|---|---|
| Lard | 20.95% |
| Bleached beeswax | 7.0% |
| Lipophilic glycerin monostearate | 2.0% |
| White petrolatum | 60.0% |
| Hinokitiol-zinc complex | 0.05% |
| Total | 100.0% |

(1) Hinokitiol-zinc complex was weighed, and a portion of lard was added thereto. The mixture was heated to about 40°C and stirred to give a homogeneous melt. (2) The remaining portion of lard, lipophilic glycerin monostearate, bleached beeswax and white petrolatum were dissolved on a water bath and stirred to give a mixture at 80°C. (3) The mixture prepared in the above step (2) was placed portionwise into a mortar with uniform stirring, and cooled to about 40°C with stirring. (4) Then, the mixture prepared in the above step (1) was added at about 40°C, and the resulting mixture was fully stirred until solidification to give the desired ointment.

### Production Example 24 (Disinfectant for pumps)

| | |
|---|---|
| Hinokitiol-zinc complex | 0.05 g |
| 99.5% Ethanol | 70.0 g |
| Purified water | Balance |
| Total | 100.0 g |

### Production Example 25 (Shampoo for pet animals)

| | |
|---|---|
| Sodium lauroyl methylalanine (30%) | 40.0 g |
| Cocoyl amidopropyldimethyl aminoacetic acid betaine | 10.0 g |
| Hinokitiol-zinc complex | 0.1 g |
| 99.5% Ethanol | 3.0 g |
| pH adjusting agent | suitable amount |
| Purified water | Balance |
| Total | 100.0 g |

### Production Example 26 (Solid soap)

| | |
|---|---|
| Soap material | 30.0 g |
| Sodium hydrogenated cocomonoglyceride sulfate | 50.0 g |
| Glyceryl monostearate | 5.0 g |
| Cetyl alcohol | 5.0 g |
| Titanium dioxide | 0.5 g |
| Hinokitiol-zinc complex | 0.05 g |
| Perfume, antioxidant and sequestering agent | suitable amount |

### Production Example 27 (Detergent for instruments)

| | |
|---|---|
| Sodium cocoyl methyl alaninate (25%) | 15.0 g |
| Cocoyl amidopropyldimethyl aminoacetic acid betaine | 5.0 g |
| Triethanolamine salt of hinokitiol-zinc complex | 0.01 g |
| L-arginine acid salt of hinokitiol-zinc complex | 0.03 g |
| pH adjusting agent | suitable amount |
| Purified water | Balance |
| Total | 100.0 g |

### Production Example 28 (Liquid detergent for laundry)

| | |
|---|---|
| Polyoxyethylene lauryl ether | 35.0 g |
| Alkylbenzenesulfonic acid triethanolamine salt | 15.0 g |
| 2-Amino-2-methyl-1,3-propanediol salt of hinokitiol-zinc complex | 0.5 g |
| Purified water | Balance |
| Total | 100.0 g |

### Production Example 29 (Liquid detergent for laundry)

| | |
|---|---|
| Alkylbenzenesulfonic acid triethanolamine salt | 19.0 g |
| Polyoxyethylene dodecyl ether | 7.0 g |
| Sodium methaxylenesulfonate | 6.0 g |
| Triethanolamine citrate | 8.0 g |
| Carboxymethylcellulose | 0.5 g |
| 2-Amino-2-methyl-1,3-propanediol salt of hinokitiol-zinc complex | 1.0 g |
| Fluorescent brightener and perfume | suitable amount |
| Purified water | Balance |
| Total | 100.0 g |

### Production Example 30 (Powdery detergent for laundry)

| | |
|---|---|
| Beef tallow soap | 45.0 g |
| Coconut oil soap | 10.0 g |
| Anhydrous sodium carbonate | 35.0 g |
| Anhydrous sodium sulfate | 7.0 g |
| Hinokitiol-zinc complex | 0.5 g |
| Sodium salt of hinokitiol-zinc complex | 0.5 g |
| Fluorescent brightener and perfume | suitable amount |
| Purified water | Balance |
| Total | 100.0 g |

### Production Example 31 (Detergent for bathroom)

| | |
|---|---|
| Polyoxyethylene alkyl (C₁₂) ether | 10.0 g |
| Citric acid | 5.0 g |
| Polypropylene glycol | 5.0 g |
| 99.5 % Ethanol | 3.0 g |
| Hinokitiol-zinc complex | 0.05 g |
| pH adjusting agent | suitable amount |
| Purified water | Balance |
| Total | 100.0 g |

### Production Example 32 (Detergent for toilet)

| | |
|---|---|
| Hydrochloric acid | 9.5 g |
| Sodium chloride | 7.0 g |
| Polyoxyethylene alkyl (C₁₂) ether | 2.0 g |
| Thickening agent | 2.5 g |
| Hinokitiol-zinc complex | 0.03 g |
| pH adjusting agent | suitable amount |
| Purified water | Balance |
| Total | 100.0 g |

### Production Example 33 (Wiping agent, bulk for aerosol)

| | |
|---|---|
| Liquid paraffin | 0.3 g |
| Sucrose fatty acid ester | 2.0 g |
| 1,3-Butylene glycol | 3.0 g |
| Hinokitiol-zinc complex | 0.02 g |
| 99.5 % Ethanol | 5.0 g |
| Purified water | Balance |
| Total | 100.0 g |

### Reference Example 2

Among the test bacterial strains used in the following test examples, MRSA is a methicillin-resistant Staphylococcus aureus strain which has, in addition to resistance to methicillin, resistance to quinolone, aminoglycoside, macrolide and tetracycline. MSSA is a methicillin-susceptible Staphylococcus aureus strain.

In respect of MRSA (clinical isolate) and MSSA (clinical isolate), the minimum inhibitory concentration (MIC) of each of hinokitiol, sodium methicillin (penicillin), Ofloxacin (quinolone), Gentamicin sulfate (aminoglycoside), Erythromycin (macrolide) and Minocycline hydrochloride (tetracycline) in a medium was determined. The results are shown in Table 1.

**Table 1**

| Result of MIC determination | | |
|---|---|---|
| | MRSA | MSSA |
| Hinokitiol | 100 | 100 |
| Sodium methicillin | >100 | 1.56 |
| Ofloxacin | 12.5 | - |
| Gentamicin sulfate | >100 | - |
| Erythromycin | >100 | - |
| Minocycline hydrochloride | 25 | - |
| Unit: µg/ml | | |

From the results shown in Table 1, strains which are resistant to a drug having a MIC of at least 12.5 are regarded as resistant to the drug. For example, MRSA is considered to be resistant to quinolone, aminoglycoside, macrolide and tetracycline, since the MICs in respect of MRSA are 12.5 µg/ml of Ofloxacin (resistance to quinolone), >100 µg/ml of Gentamicin sulfate (resistance to aminoglycoside), >100 µg/ml of Erythromycin (resistance to macrolide) and 25 µg /ml of Minocycline hydrochloride (resistance to tetracycline).

### Test Example 1 (Antibacterial activity test)

The drug tested was hinokitiol-copper complex. The bacterial strain used was Staphylococcus (S.) aureus 209PJC.

An ethanol solution containing 0.8% of hinokitiol-copper complex was prepared, and serially double-diluted with a 50% ethanol solution to prepare solutions containing hinokitiol-copper complex in various concentrations.

The media used were Trypto Soy broth medium as the preculture medium, broth medium for dilution, and heart infusion agar medium for plate preparation. The incubation temperature was 37°C and the incubation period was 20 hours.

The MIC determined in a conventional manner is shown in Table 2.

**Table 2**

| Bacterial strain | MIC (µg/ml) |
|---|---|
| S. aureus 209 PJC | 0.78 |

### Test Example 2 (Antibacterial activity test)

The effects of the combined use of hinokitiol and a copper compound were examined by the checkerboard dilution method.

The drugs tested were as follows. Hinokitiol: Hinokitiol SP, product of Takasago Perfumery
Co., Ltd., Lot. No. 112108
Copper (I) chloride: product of Nacalai Kagaku Yakuhin K.K., Lot. No. M3G4518
Copper (I) sulfate pentahydrate: product of Nacalai Kagaku Yakuhin K.K., Lot. No. M35740
Copper (II) oxide: product of Nacalai Kagaku Yakuhin K.K., Lot. No. M3F3876
The bacterial strains used were as follows. S. aureus 209PJC
S. epidermidis ATCC14990
MRSA1 (clinical isolate)
MSSA1 (clinical isolate, methicillin-susceptible Staphylococcus aureus)
Cells of the above strains were precultured in Trypto Soy broth medium (incubation temperature: 37°C; incubation period: 18 to 20 hours) and the cultures were diluted with broth medium to 10⁶ cells/ml, and the dilutions were used as the test bacterial cell suspensions.

The concentrations of the drugs for testing and the method of preparing the drugs were as follows. Thus, an alcohol solution containing 5% of hinokitiol was diluted with sterile distilled water to give a 0.4% hinokitiol solution. This solution was serially double-diluted with sterile distilled water to give solutions containing hinokitiol in the concentrations of 2000 µg/ml, 1000 µg/ml, 500 µg/ml, 250 µg/ml, 125 µg/ml, 62.5 µg/ml, 31.3 µg/ml, 15.6 µg/ml, 7.8 µg/ml and 3.9 µg/ml. When copper (I) chloride or copper (I) sulfate pentahydrate was to be used as the copper compound, an aqueous solution containing 3.2% of the copper compound was serially double-diluted with sterile distilled water to give aqueous solutions containing the copper compound in the concentrations of 16000 µg/ml, 8000 µg/ml, 4000 µg/ml, 2000 µg/ml, 1000 µg/ml, 500 µg/ml, 250 µg/ml, 125 µg/ml, 62.5 µg/ml and 31.3 µg/ml. When copper (II) oxide was to be used as the copper compound, a dispersion of 3.2% of the copper compound in sterile distilled water was serially double-diluted with sterile distilled water to give dispersions containing the copper compound in the concentrations of 16000 µg/ml, 8000 µg/ml, 4000 µg/ml, 2000 µg/ml, 1000 µg/ml, 500 µg/ml, 250 µg/ml. 125 µg/ml, 62.5 µg/ml and 31.3 µg/ml.

When hinokitiol and a copper compound were to be used combinedly, 0.5 ml each of the above two drug solutions having respectively 20 times the concentrations used were placed, together with 9 ml of the medium in each petri dish to obtain an agar plate. When hinokitiol and the copper compound were to be used individually, 1 ml of a 10-fold concentrated solution of hinokitiol or the copper compound and 9 ml of the medium were placed in each petri dish to obtain an agar plate.

Heart infusion agar plates containing hinokitiol and/or copper compound at various concentrations were prepared by the above method and inoculated with each bacterial cell suspension with the cell concentration of 10⁶ cells/ml and, after incubating at 37°C for 20 hours, judgement was made as to whether cells had grown or not. The results obtained are shown in Tables 3 to 14. In the following tables, "+" means that growth was observed, "-" means that no growth was observed, and "±" means that very slight growth was observed.

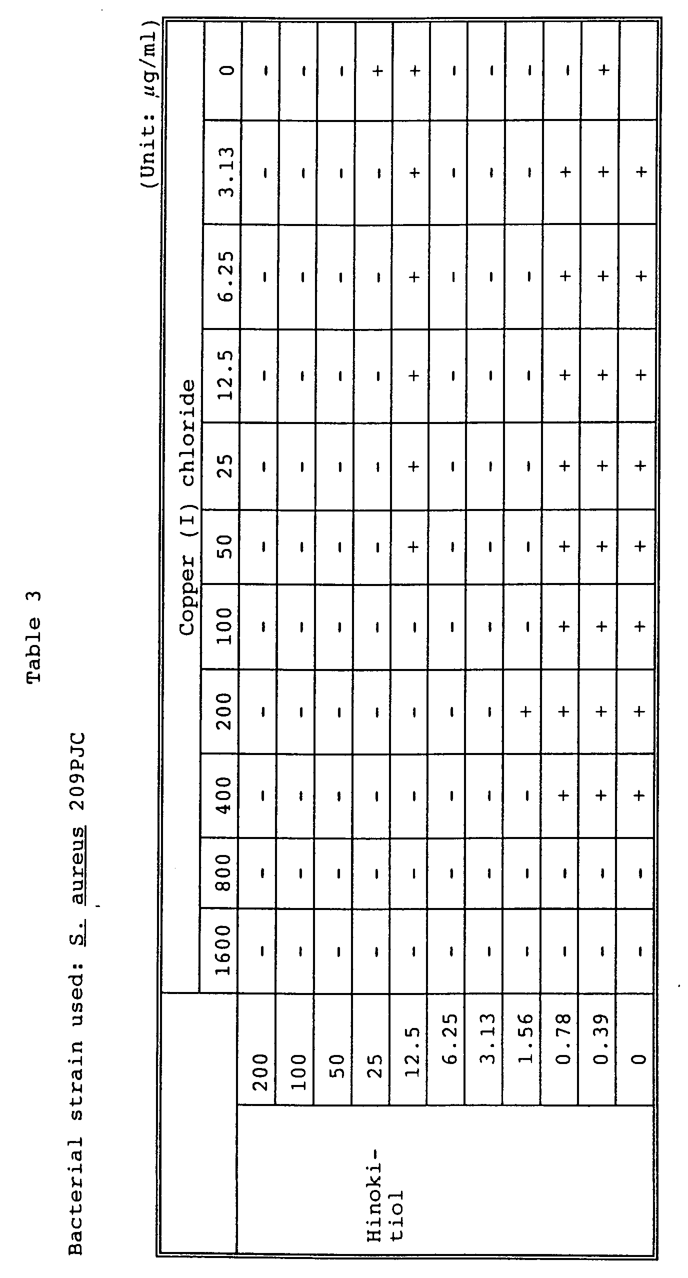

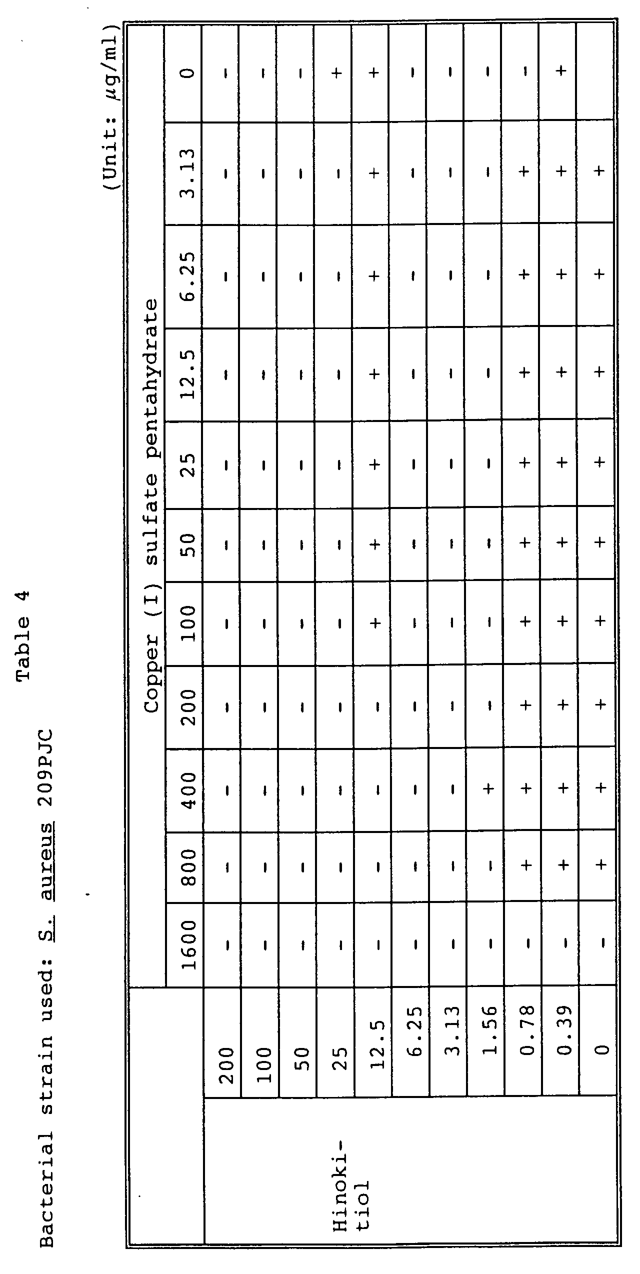

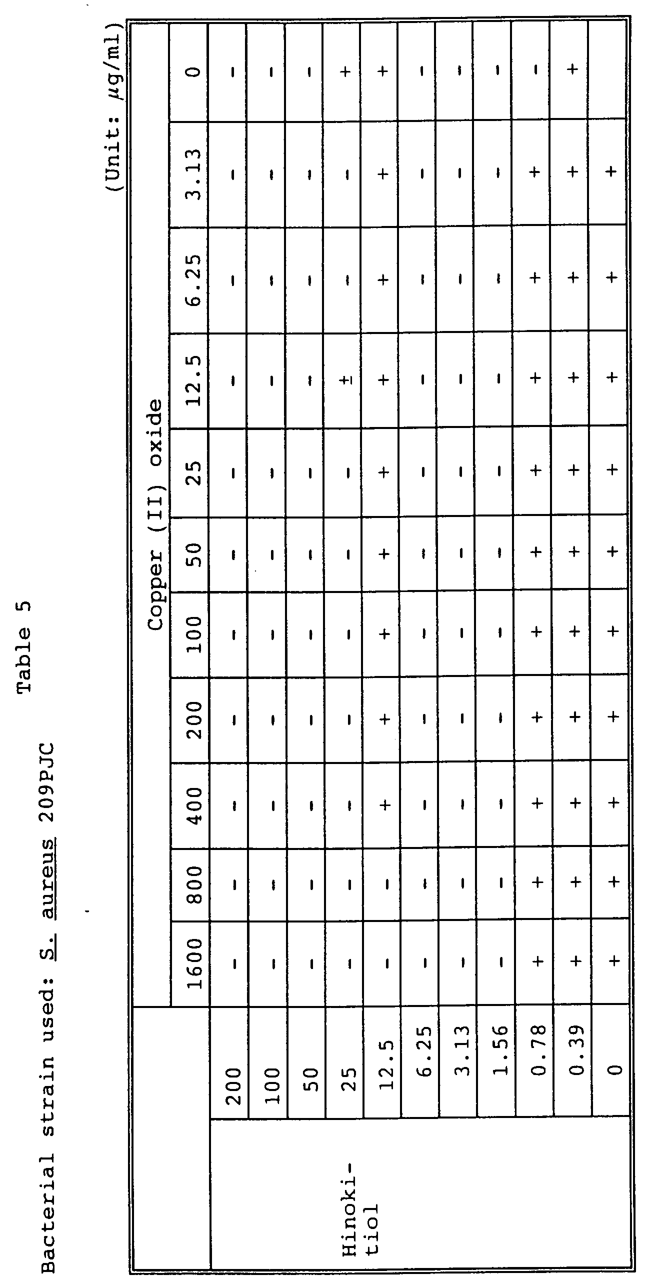

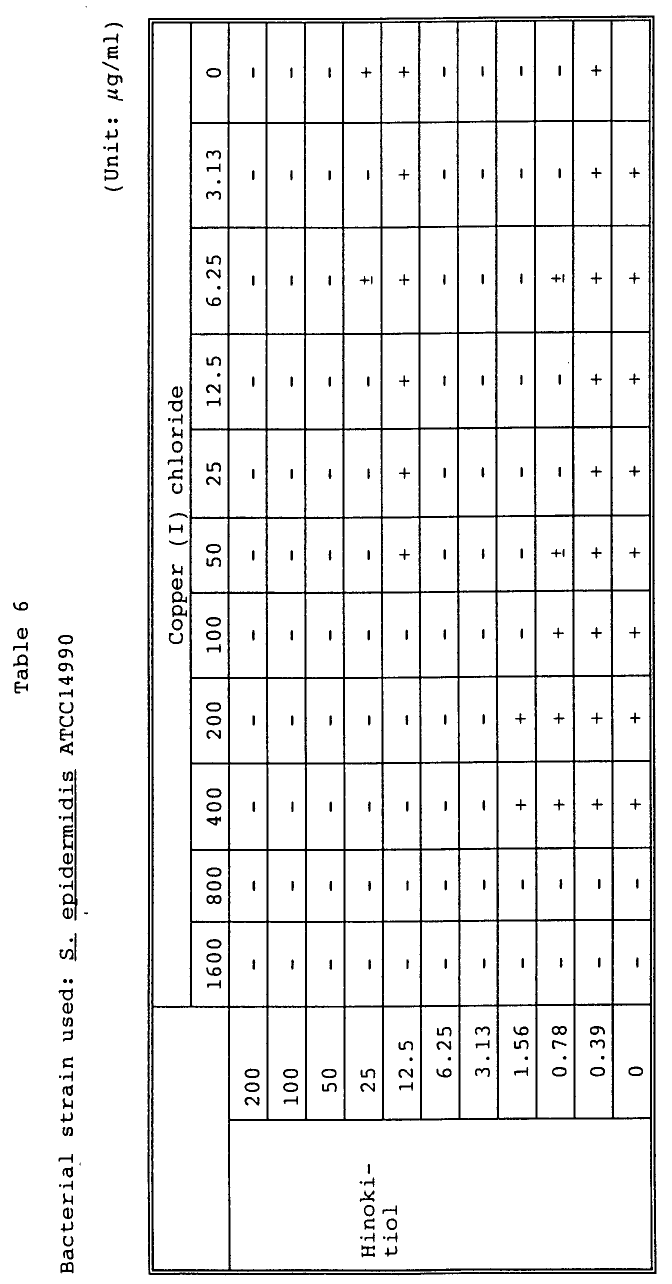

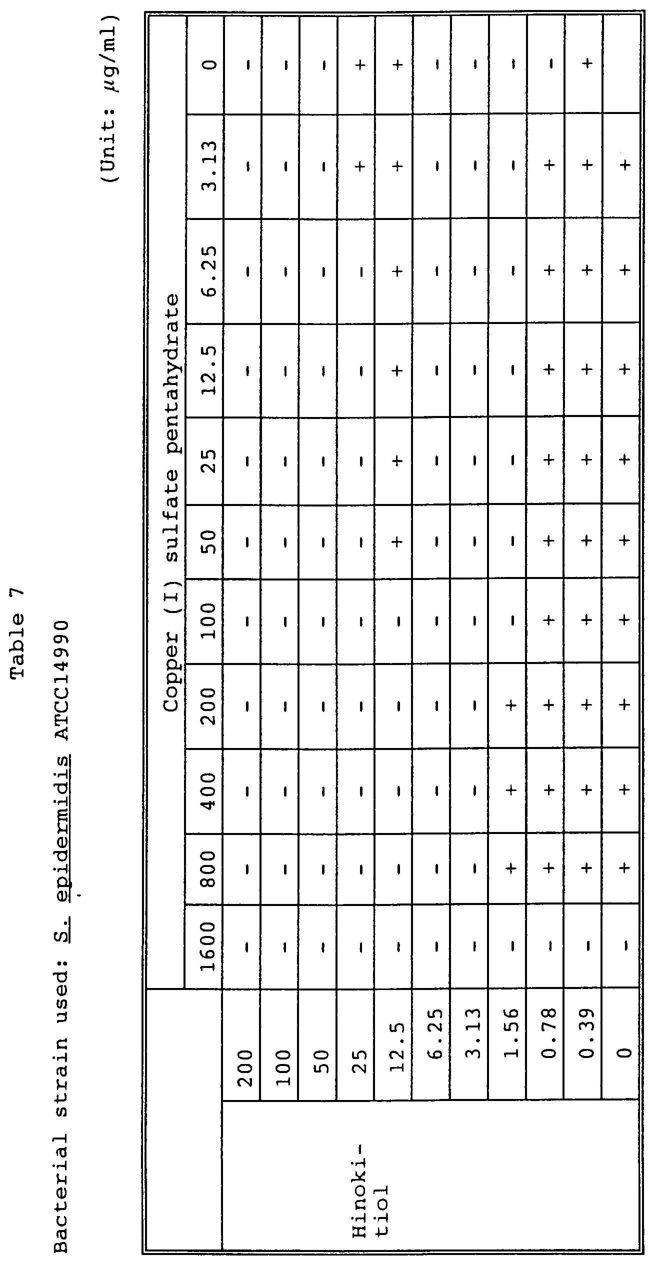

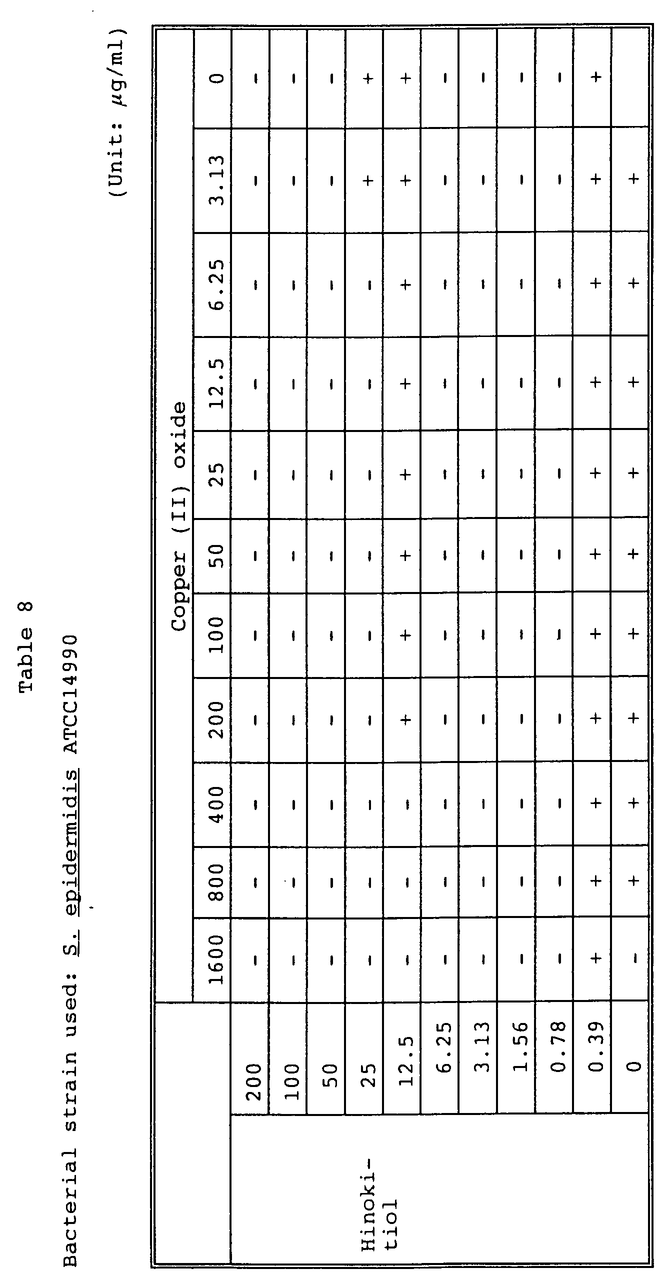

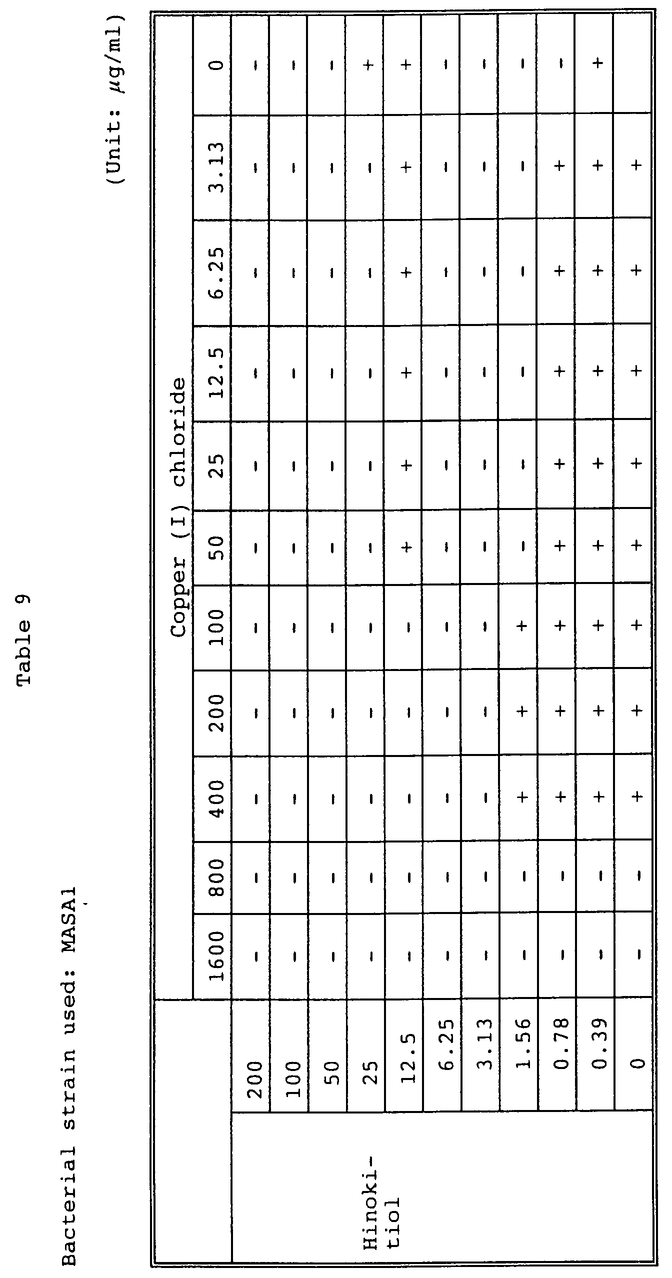

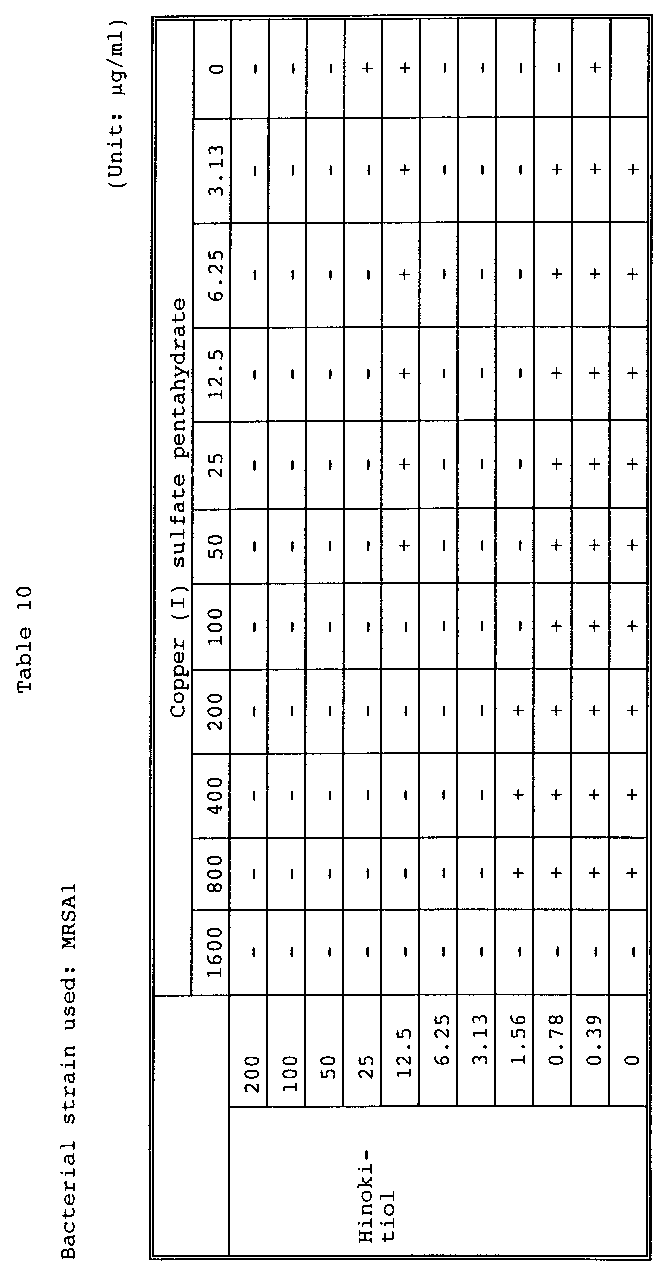

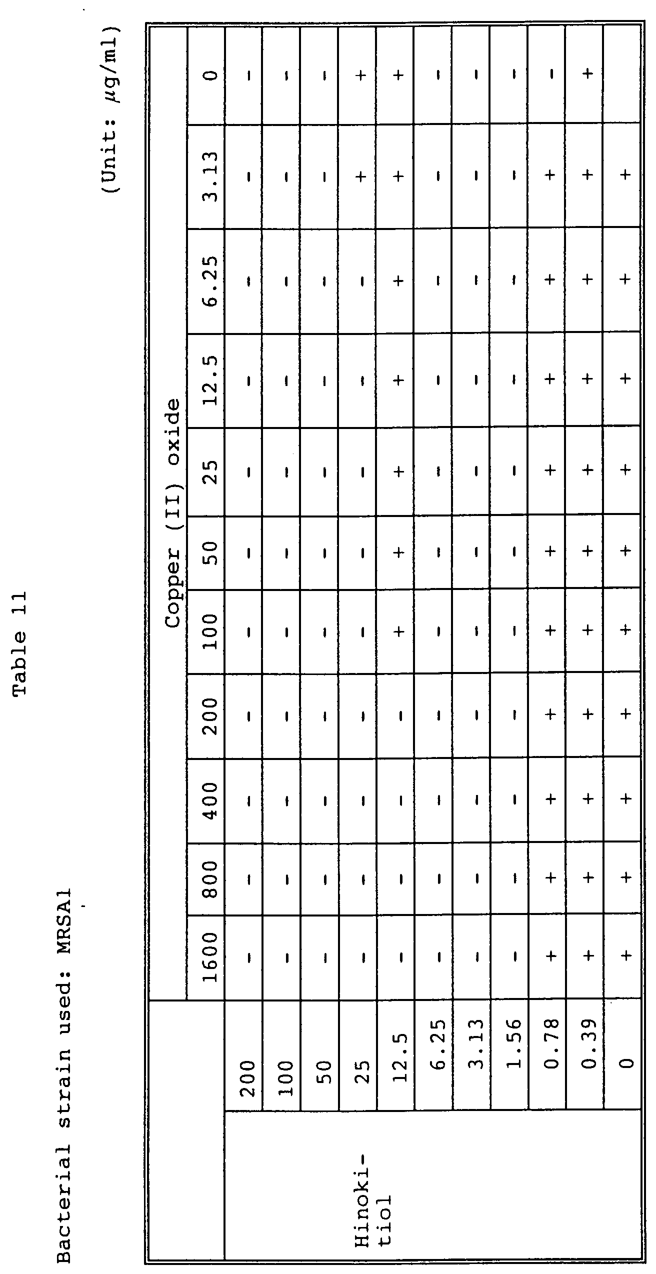

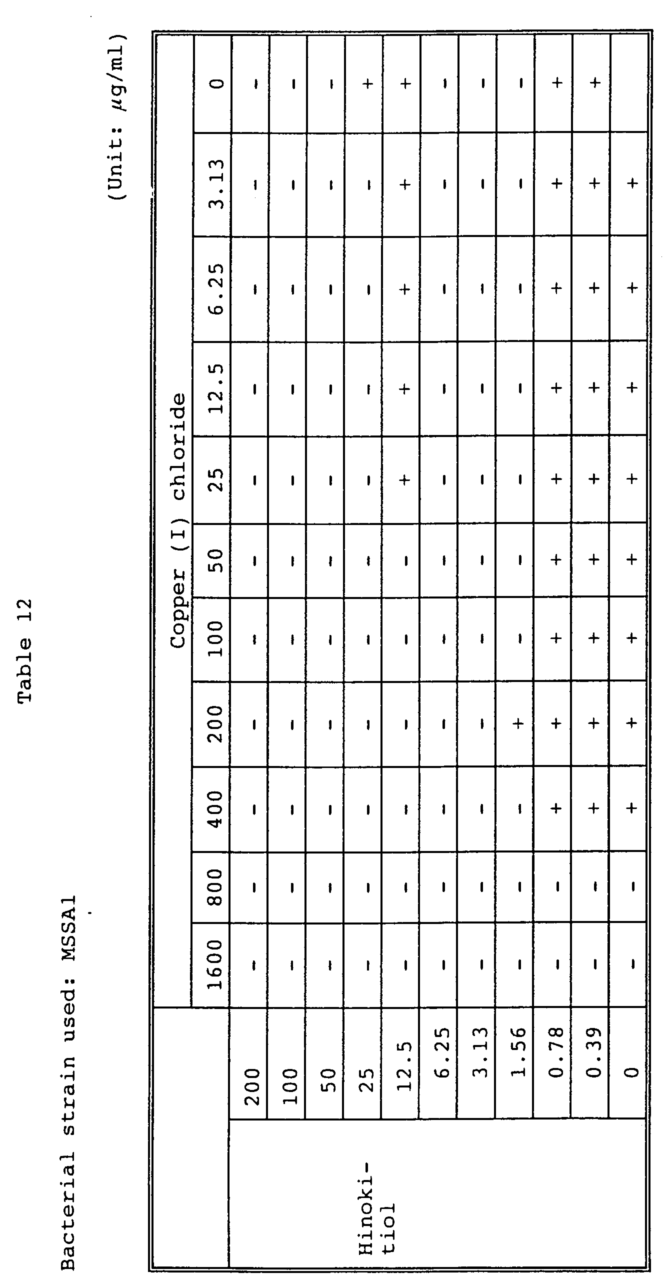

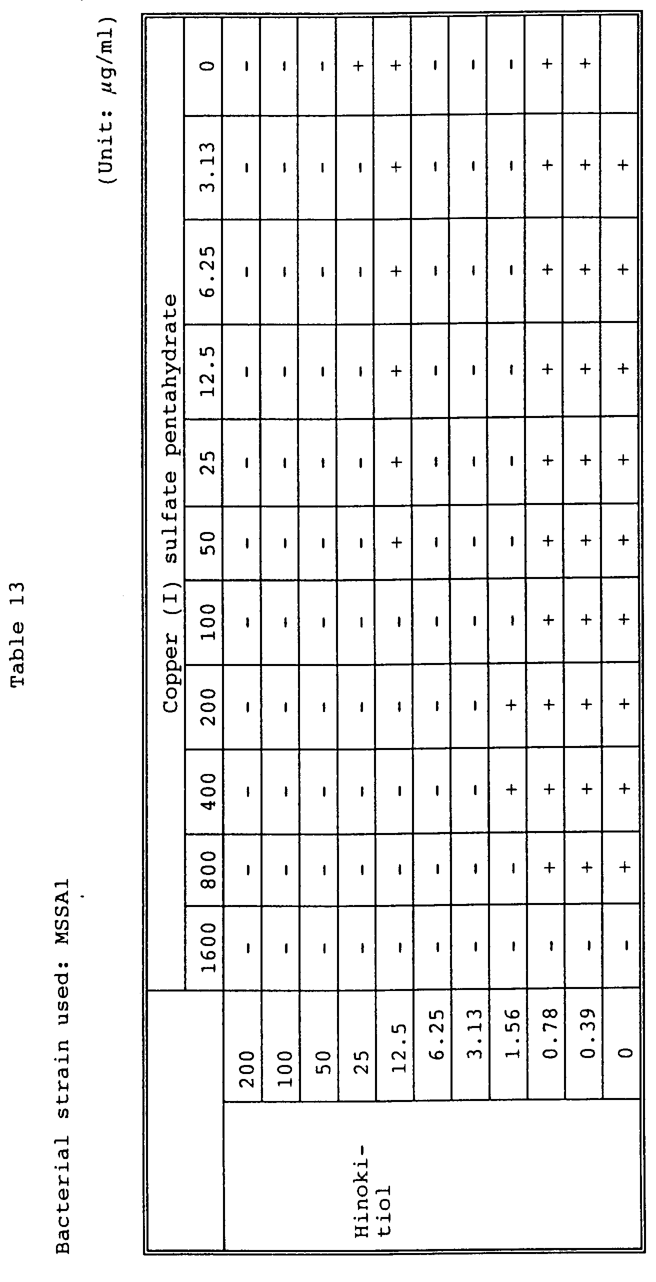

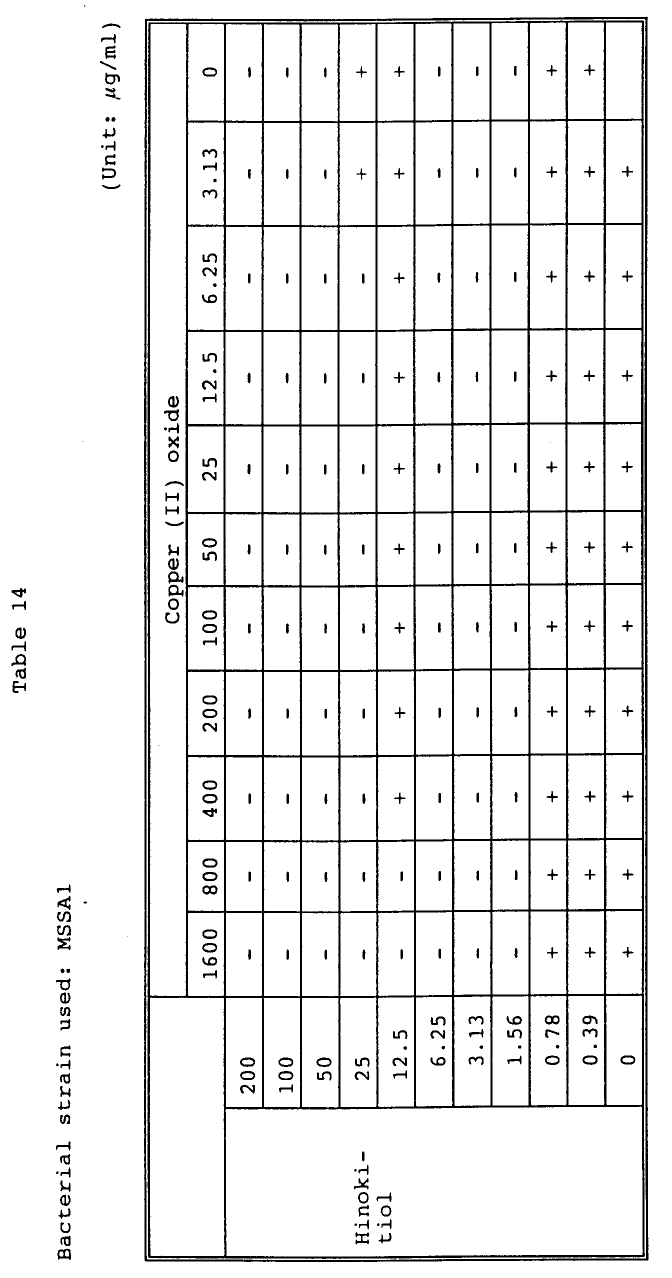

As seen from the results shown in Tables 3 to 14, the use of hinokitiol in combination with a copper compound remarkably improves the antimicrobial activity. Test Example 3 (Antibacterial activity test)

The drug tested was the hinokitiol-zinc complex obtained in the above Reference Example. The bacterial strain used was Staphylococcus (S.) aureus 209PJC.

An ethanol solution containing 0.8% of hinokitiol-zinc complex was prepared, and serially double-diluted with a 50% ethanol solution to prepare solutions containing hinokitiol-zinc complex in various concentrations.

The media used were Trypto Soy broth medium as the preculture medium, broth medium for dilution, and heart infusion agar medium for plate preparation. The incubation temperature was 37°C and the incubation period was 20 hours.

The MIC determined in a conventional manner is shown in Table 15.

**Table 15**

| Bacterial strain | MIC (µg/ml) |
|---|---|
| S. aureus 209PJC | 50 |

### Test Example 4 (Antibacterial activity test)

The drug tested was the hinokitiol-zinc complex obtained in the above Reference Example. The bacterial strain used was Pityrosporum ovale IFO 0656.

An ethanol solution containing 0.8% of hinokitiol-zinc complex was prepared, and serially double-diluted with a 50% ethanol solution to prepare solutions containing hinokitiol-zinc complex in various concentrations.

The media used were a potato dextrose slant medium containing 5% of polyoxyethylene (5) glyceryl monooleate as the preculture medium, saline solution containing 0.7% of polyoxyethylene (20) sorbitan monooleate for dilution, and a potato dextrose agar medium containing 5% of polyoxyethylene (5) glyceryl monooleate for plate preparation. The incubation temperature was 38°C and the incubation period was 48 hours.

The MIC determined in a conventional manner is shown in Table 16.

**Table 16**

| Bacterial strain | MIC (µg/ml) |
|---|---|
| P. ovale IFO 0656 | 12.5 |

### Test Example 5 (Antibacterial activity test)

The drugs tested were hinokitiolato copper and hinokitiol. The bacterial strain used was Staphylococcus (S.) aureus 209PJC. The test bacterial strain precultured to the logarithmic growth phase in Trypto Soy broth medium (18 to 20 hours, 37°C) were suspended in heart infusion broth medium. Drug solutions which had been adjusted to predetermined drug concentrations were allowed to act on the test bacterial cell suspension. Then, shaking culture was performed at 37°C and viable cells were counted at timed intervals (diluent: SCDLP medium; medium: SCDLP agar medium; incubation period: 24 to 48 hours).

The method of preparing the drug solutions was as follows. When hinokitiol is to be used, an ethanol solution containing 2% of hinokitiol (product of Takasago Perfumery Co., Ltd.) was diluted with sterile distilled water to give solutions containing hinokitiol in 10 and 20 times the concentration of the drug solutions to be used. When hinokitiolato copper is to be used, an ethanol solution containing 2% of hinokitiolato copper (product of Takasago Perfumery Co., Ltd.) was prepared and diluted with sterile distilled water to give solutions containing hinokitiolato copper in 10 and 20 times the concentrations of the drug solutions to be used. When hinokitiol or hinokitiolato copper were to be used each individually, 7.2 ml of the bacterial cell suspension was added to 0.8 ml of the 10-fold concentrated hinokitiol or hinokitiolato copper solution. When hinokitiol and hinokitiolato copper were to be used combinedly, 7.2 ml of the bacterial cell suspension was added to a mixture of 0.4 ml each of the 20-fold concentrated hinokitiol and hinokitiolato copper solutions. A control was prepared by adding 7.2 ml of the cell suspension to 0.8 ml of sterile distilled water.

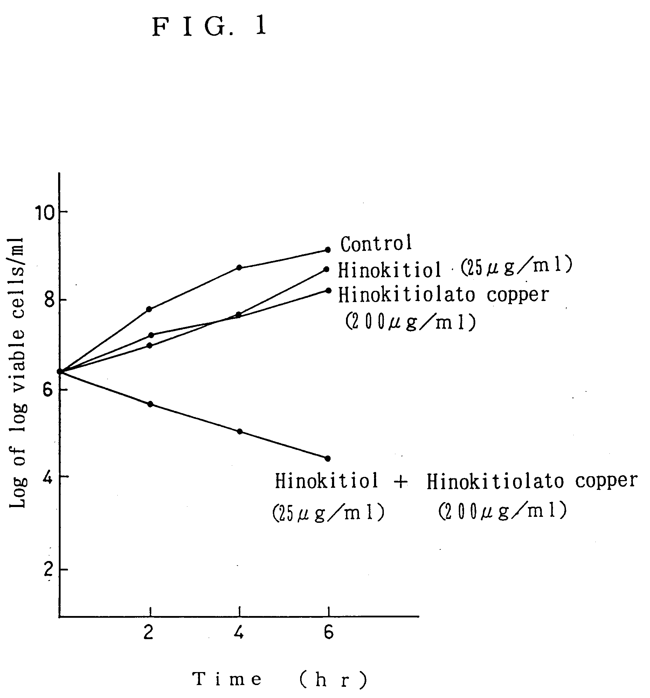

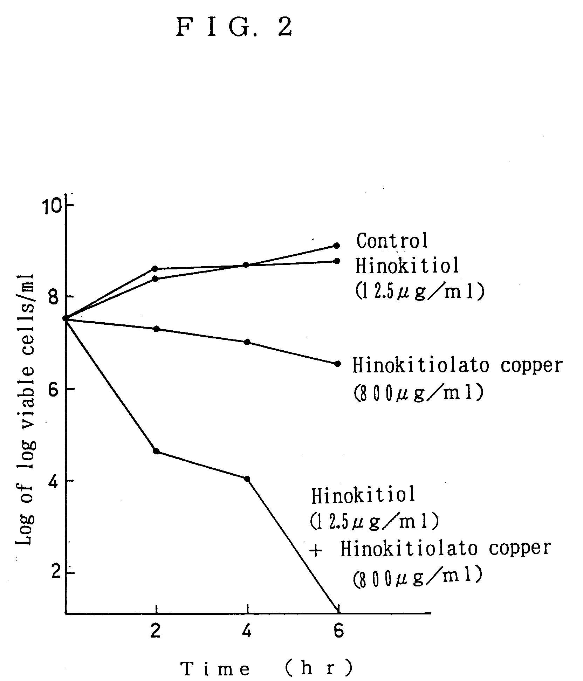

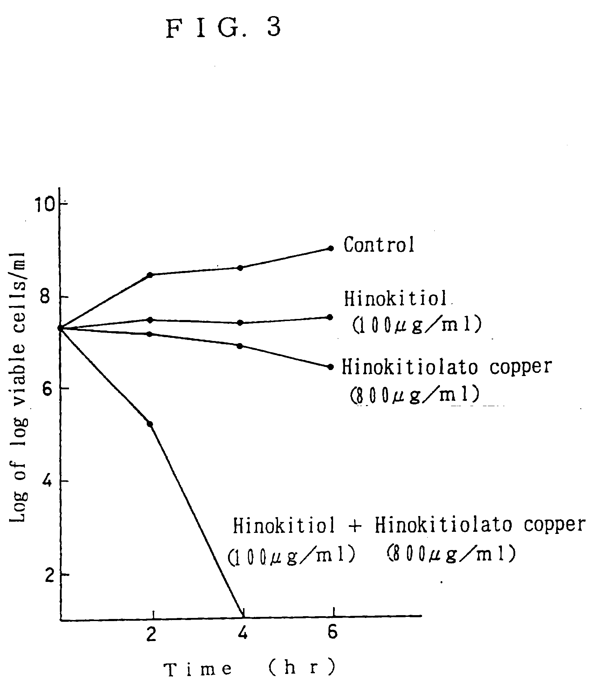

Viable cells were counted at timed intervals and the results are shown in Figs. 1 to 3.

As apparent from Figs. 1 to 3, which are graphs showing the change in the number of viable cells counted at timed intervals, the antibacterial activity is remarkably improved by using hinokitiol and hinokitiolato copper in combination.

### BRIEF DESCRIPTION OF THE DRAWINGS

Figs. 1, 2 and 3 are graphs showing the change in the number of viable cells counted at timed intervals.

## Claims

1. An antimicrobial method comprising administering to a patient at least one member selected from hinokitiol-copper complexes and salts thereof, or at least one member selected from hinokitiol-zinc complexes and salts thereof.

2. A method as claimed in claim 1 wherein the microorganism is a microorganism other than fungi.

3. An antimicrobial method comprising administering to a patient a copper compound and at least one member selected from hinokitiol and salts thereof.

4. A method as claimed in claim 3 wherein the microorganism is a microorganism other than fungi.

5. A method as claimed in claim 3 wherein said copper compound and said at least one member selected from hinokitiol and salts thereof are administered in a total amount of about 1 to 20 mg/kg body weight in one to three divided doses.

6. A method as claimed in claim 4 wherein said copper compound and said at least one member selected from hinokitiol and salts thereof are administered in a total amount of about 1 to 20 mg/kg body weight in one to three divided dose.

7. A method as claimed in claim 5 or 6 wherein based on the total amount of said copper compound and said at least one member selected from hinokitiol and salts thereof, said copper compound is used in an amount of about 0.05 to 99.95% by weight and said at least one member selected from hinokitiol and salts thereof is used in an amount of about 99.95 to 0.05% by weight.

8. A method as claimed in claim 7 wherein based on the total amount of said copper compound and said at least one member selected from hinokitiol and salts thereof, said copper compound is used in an amount of about 10 to 99.95% by weight and said at least one member selected from hinokitiol and salts thereof is used in an amount of about 90 to 0.05% by weight.

9. A method as claimed in claim 8 wherein based on the total amount of said copper compound and said at least one member selected from hinokitiol and salts thereof, said copper compound is used in an amount of about 50 to 99.9% by weight and said at least one member selected from hinokitiol and salts thereof is used in an amount of about 50 to 0.1% by weight.

10. A method as claimed in claim 3, 4, 5, 6, 8 or 9 wherein said copper compound is at least one member selected from the group consisting of copper (I) chloride, copper (II) acetate, copper (II) ammonium chloride, copper (I) bromide, copper (II) bromide, copper (I) bromide-dimethyl sulfide complex, copper (II) carbonate, copper (II) citrate, copper (I) cyanide, copper 4-cyclohexylbutyrate, copper (II) diammonium chloride, copper (II) diphosphate, copper (II) fluoride, copper (II) formate, copper (II) gluconate, copper (II) hydroxide, copper (I) iodide, copper naphthenate, copper (II) nitrate, copper (II) oleate, copper (II) oxalate, copper (II) oxide, copper (II) phosphate, copper (II) phthalate, copper (II) potassium chloride, copper (I) rhodanide, copper (II) sulfate, copper (II) sulfide, copper (I) thiocyanate, copper chlorophyll, hinokitiolato copper, copper nicotinate, nicotinamide copper compound, copper picolinate, picolinamide copper compound and copper salts and copper complexes of a compound selected from amino acids and peptides.

11. A method as claimed in claim 3, 4, 5, 6, 8 or 9 wherein said copper compound is at least one member selected from the group consisting of copper (I) chloride, copper (II) acetate, copper (II) ammonium chloride, copper (I) bromide, copper (II) bromide, copper (I) bromide-dimethyl sulfide complex, copper (II) carbonate, copper (II) citrate, copper (I) cyanide, copper 4-cyclohexylbutyrate, copper (II) diammonium chloride, copper (II) diphosphate, copper (II) fluoride, copper (II) formate, copper (II) gluconate, copper (II) hydroxide, copper (I) iodide, copper naphthenate, copper (II) nitrate, copper (II) oleate, copper (II) oxalate, copper (II) oxide, copper (II) phosphate, copper (II) phthalate, copper (II) potassium chloride, copper (I) rhodanide, copper (II) sulfate, copper (II) sulfide, copper (I) thiocyanate, hinokitiolato copper, copper nicotinate, nicotinamide copper compound, copper picolinate, picolinamide copper compound and copper salts and copper complexes of a compound selected from amino acids and peptides.

12. A method as claimed in claim 3, 4, 5, 6, 8 or 9 wherein the copper compound is at least one member selected from the group consisting of copper (I) chloride, copper (II) acetate, copper (II) citrate, copper (II) gluconate, copper (II) oxide, copper (II) sulfate and hinokitiolato copper.

13. A method as claimed in claim 3, 4, 5, 6, 8 or 9 wherein the copper compound is at least one member selected from the group consisting of copper (I) chloride, copper (II) oxide, copper (II) sulfate and hinokitiolato copper.

14. A method as claimed in claim 3 for treating an infectious disease caused by microorganisms in a patient, the method comprising administering to said patient a copper compound and at least one member selected from hinokitiol and salts thereof, said copper compound being at least one member selected from the group consisting of copper (I) chloride, copper (II) acetate, copper (II) ammonium chloride, copper (I) bromide, copper (II) bromide, copper (I) bromide-dimethyl sulfide complex, copper (II) carbonate, copper (II) citrate, copper (I) cyanide, copper 4-cyclohexylbutyrate, copper (II) diammonium chloride, copper (II) diphosphate, copper (II) fluoride, copper (II) formate, copper (II) gluconate, copper (II) hydroxide, copper (I) iodide, copper naphthenate, copper (II) nitrate, copper (II) oleate, copper (II) oxalate, copper (II) oxide, copper (II) phosphate, copper (II) phthalate, copper (II) potassium chloride, copper (I) rhodanide, copper (II) sulfate, copper (II) sulfide, copper (I) thiocyanate, hinokitiolato copper, copper nicotinate, nicotinamide copper compound, copper picolinate, picolinamide copper compound and copper salts and copper complexes of a compound selected from amino acids and peptides.

15. A method as claimed in claim 14 wherein the copper compound is at least one member selected from the group consisting of copper (I) chloride, copper (II) oxide, copper (II) sulfate and hinokitiolato copper.

16. A cosmetic composition comprising:
at least one copper compound selected from the group consisting of copper (I) chloride, copper (II) acetate, copper (II) ammonium chloride, copper (I) bromide, copper (II) bromide, copper (I) bromide-dimethyl sulfide complex, copper (II) carbonate, copper (II) citrate, copper (I) cyanide, copper 4-cyclohexylbutyrate, copper (II) diammonium chloride, copper (II) diphosphate, copper (II) fluoride, copper (II) formate, copper (II) gluconate, copper (II) hydroxide, copper (I) iodide, copper naphthenate, copper (II) nitrate, copper (II) oleate, copper (II) oxalate, copper (II) oxide, copper (II) phosphate, copper (II) phthalate, copper (II) potassium chloride, copper (I) rhodanide, copper (II) sulfate, copper (II) sulfide, copper (I) thiocyanate, hinokitiolato copper, copper nicotinate, nicotinamide copper compound, copper picolinate, picolinamide copper compound and copper salts and copper complexes of a compound selected from amino acids and peptides, and
at least one member selected from hinokitiol and salts thereof.

17. A cosmetic composition as claimed in claim 16 wherein said copper compound and said at least one member selected from hinokitiol and salts thereof are used in a total amount of about 0.0001 to 99.9% by weight based on the amount of said composition.

18. A cosmetic composition as claimed in claim 16 wherein said copper compound and said at least one member selected from hinokitiol and salts thereof are used in a total amount of about 0.001 to 30% by weight based on the amount of said composition.

19. A cosmetic composition as claimed in claim 18 wherein based on the total amount of said copper compound and said at least one member selected from hinokitiol and salts thereof, said copper compound is used in an amount of about 0.05 to 99.95% by weight and said at least one member selected from hinokitiol and salts thereof is used in an amount of about 99.95 to 0.05% by weight.

20. A cosmetic composition as claimed in claim 19 wherein based on the total amount of said copper compound and said at least one member selected from hinokitiol and salts thereof, said copper compound is used in an amount of about 10 to 99.95% by weight and said at least one member selected from hinokitiol and salts thereof is used in an amount of about 90 to 0.05% by weight.

21. A cosmetic composition as claimed in claim 20 wherein based on the total amount of said copper compound and said at least one member selected from hinokitiol and salts thereof, said copper compound is used in an amount of about 50 to 99.9% by weight, and said at least one member selected from hinokitiol and salts thereof is used in an amount of about 50 to 0.1% by weight.

22. A cosmetic composition as claimed in any one of claims 16 to 21 wherein said copper compound is at least one member selected from the group consisting of copper (I) chloride, copper (II) acetate, copper (II) citrate, copper (II) gluconate, copper (II) oxide, copper (II) sulfate and hinokitiolato copper.

23. A cosmetic composition as claimed in any one of claims 16 to 21 wherein said copper compound is at least one member selected from the group consisting of copper (I) chloride, copper (II) oxide, copper (II) sulfate and hinokitiolato copper.

24. A method of preserving cosmetics using:
at least one copper compound selected from the group consisting of copper (I) chloride, copper (II) acetate, copper (II) ammonium chloride, copper (I) bromide, copper (II) bromide, copper (I) bromide-dimethyl sulfide complex, copper (II) carbonate, copper (II) citrate, copper (I) cyanide, copper 4-cyclohexylbutyrate, copper (II) diammonium chloride, copper (II) diphosphate, copper (II) fluoride, copper (II) formate, copper (II) gluconate, copper (II) hydroxide, copper (I) iodide, copper naphthenate, copper (II) nitrate, copper (II) oleate, copper (II) oxalate, copper (II) oxide, copper (II) phosphate, copper (II) phthalate, copper (II) potassium chloride, copper (I) rhodanide, copper (II) sulfate, copper (II) sulfide, copper (I) thiocyanate, hinokitiolato copper, copper nicotinate, nicotinamide copper compound, copper picolinate, picolinamide copper compound and copper salts and copper complexes of a compound selected from amino acids and peptides, and
at least one member selected from hinokitiol and salts thereof.

25. A method for preserving cosmetics as claimed in claim 24 wherein said copper compound and said at least one member selected from hinokitiol and salts thereof are used in a total amount of about 0.0001 to 99.9% by weight based on the amount of said composition.

26. A method for preserving cosmetics as claimed in claim 25 wherein said copper compound and said at least one member selected from hinokitiol and salts thereof are used in a total amount of about 0.001 to 30% by weight based on the amount of said composition.

27. A method for preserving cosmetics as claimed in claim 26 wherein based on the total amount of said copper compound and said at least one member selected from hinokitiol and salts thereof, said copper compound is used in an amount of about 50 to 99.9% by weight, and said at least one member selected from hinokitiol and salts thereof is used in an amount of about 50 to 0.1% by weight.

28. A method for preserving cosmetics as claimed in any one of claims 24 to 27 wherein said copper compound is at least one member selected from the group consisting of copper (I) chloride, copper (II) acetate, copper (II) citrate, copper (II) gluconate, copper (II) oxide, copper (II) sulfate and hinokitiolato copper.

29. A method for preserving cosmetics as claimed in any one of claims 24 to 27 wherein said copper compound is at least one member selected from the group consisting of copper (I) chloride, copper (II) oxide, copper (II) sulfate and hinokitiolato copper.
